# EUROPEAN PATENT APPLICATION

(11) **EP 1 832 265 A2**
(43) Date of publication of application: **12.09.2007**
(21) Application number: 07012861.6
(22) Date of filing: 19.06.2003
(51) Int. Cl.: A61F 13/26

(54) **Tampon applicator with improved fingergrip and method of making same**

(30) Priority: 21.06.2002 US 390309 P; 17.06.2003 US 463202
(62) Divisional of application: 03761361.9
(71) Applicant: PLAYTEX PRODUCTS, INC., Westport, CT 06880 (US)
(72) Inventor: Miller, Michael L., Dover DE 19904 (US); Melvin, Wayne D., Camdem DE 19943 (US)
(74) Representative: Weitzel, Wolfgang

(57) **Abstract**

The present invention provides a tampon applicator barrel having a fingergrip with one or more gripping structures that have an acute edge for improved gripping. Preferably, the one or more gripping structures are one or more embossed ring-like structures. The present invention also provides a method for forming the one or more gripping structures on the applicator barrel. The method preferably includes forming at least one embossed ring-like gripping structure on the barrel such that the radial compressive strength of the applicator barrel is not compromised.

## Description

### BACKGROUND OF THE INVENTION

### 1. Field of the Invention

The present invention relates generally to a catamenial device or applicator with an improved fingergrip. More particularly, the present invention relates to an applicator with an improved embossed fingergrip and a method for making the improved embossed fingergrip.

### 2. Description of the Prior Art

A catamenial insertion device or applicator normally has two components, namely a barrel and a plunger that is adapted to telescopically slide in the barrel. The material to be expelled, such as an absorbent pledget, is positioned in the barrel of the applicator. The barrel has a first end for ejection of the pledget, and a second end for receipt of the plunger. To use the tampon applicator, the consumer will grasp the barrel near the second end, position the ejection end appropriately, insert the majority of the barrel into the vaginal canal, and move or slide the plunger in the barrel towards the ejection end of the barrel to expel the pledget.

Tampon pledgets, and notably radially expanding pledgets, due to their design, exert a pressure or friction force on the inside wall of the applicator barrel. Thus, expulsion of the pledget from the barrel requires an applicator with a gripping configuration conducive to secure holding by the user with minimal pressure being applied to the barrel. The significance of minimizing pressure on the barrel of the applicator is that deformation of the barrel is reduced. Such barrel deformation causes significant friction amongst the pledget, barrel, and plunger, thereby significantly impeding the expulsion of the pledget from the barrel.

Various configurations for fingergrip areas on the barrel of an applicator have been proposed to facilitate handling and placement of the applicator, and expulsion of the pledget. One approach is a tampon applicator having a fingergrip that is formed by embossing an outside surface of the barrel of the applicator.

Fingergrips on cardboard tampon applicators, when embossed, are generally embossed into the cardboard from the inside so that raised projections are formed on the outside surface. These projections usually have highly curved or highly angled side walls and do not present an acute edge for secure gripping. Alternatively, holes formed either completely or partially through the fingergrip area of the applicator barrel can create an acute edge for gripping. However, this results in substantially weakening the radial compressive strength of the applicator barrel. Therefore, there exists a risk of the user compressing the applicator barrel while holding the barrel, resulting in additional resistance to the pledget during expulsion from the applicator barrel.

U.S. Patent No. 5,558,631 to Campion et al. discloses a paper laminate applicator with embossed rings. The embossed rings have a radius of curvature less than 0.060 inches. Campion also discloses that attempting such embossed formations in conventional paper applications would tend to rip, shear or otherwise damage the paper.

U.S. Patent No. 5,709,652 to Hagerty discloses a tampon applicator with apertures provided in the barrel to provide the acute edge for gripping. However, as noted above, this is known to weaken the radial compressive strength of the fingergrip region of the barrel, which could result in making ejection of the pledget more difficult if the user squeezes the grip too hard and it collapses.

Therefore, there is a need in the art for an embossed fingergrip that not only provides an acute edge for improved gripping, but also does not compromise the radial compressive strength of the barrel. The present invention provides at least one embossed gripping structure with at least one acute gripping edge that not only does not compromise the radial compressive strength of the barrel, but may actually increase the radial compressive strength of the barrel. Also, a novel method of forming such an embossed fingergrip structure is disclosed.

### SUMMARY OF THE INVENTION

It is an object of the present invention to provide a tampon applicator barrel with an improved fingergrip.

It is another object of the present invention to provide such a tampon applicator barrel with a fingergrip having one or more embossed gripping structures.

It is still another object of the present invention to provide such a fingergrip with one or more embossed gripping structures having at least one acute edge for improved gripping.

It is a further object of the present invention to provide a method for forming a tampon applicator barrel with a fingergrip having one or more embossed gripping structures with acute edges for gripping.

It is still a further object of the present invention to provide such a method for forming both the one or more embossed gripping structures and a substantially closed petal tip applicator barrel substantially simultaneously.

It is yet a further object of the present invention to provide such a method that maintains and/or improves the radial compressive strength of the tampon applicator barrel.

The above and other objects and advantages of the present invention are provided by a tampon applicator barrel having a fingergrip with one or more gripping structures that have at least one acute edge for improved gripping. Preferably, the one or more gripping structures are one or more embossed, raised, ring-like structures. The present invention also provides a method for forming the one or more gripping structures on the applicator barrel. The method preferably includes forming at least one embossed ring-like structure on the barrel such that the radial compressive strength of the applicator barrel is not compromised.

### BRIEF DESCRIPTION OF THE DRAWINGS

Fig. 1 is a plane view of one embodiment of a tampon applicator according to the present invention;
Fig. 2 is an enlarged view of the applicator barrel and gripping structures of the present invention;
Fig. 3 is a cross-sectional view of the gripping structure taken along line A-A in Fig. 2;
Fig. 4 is a plan view of another embodiment of a tampon applicator of the present invention;
Fig. 5 is a plan view of a tampon applicator barrel of the present invention positioned on a mandrel for embossing;
Fig. 6 is a plan view of a tampon applicator barrel with individual embossments formed according to one aspect of the present invention; and
Fig. 7 is a plan view of a tampon applicator barrel prior to forming a petal-tip and embossed ring-like structures according to the present invention.

### DETAILED DESCRIPTION OF THE INVENTION

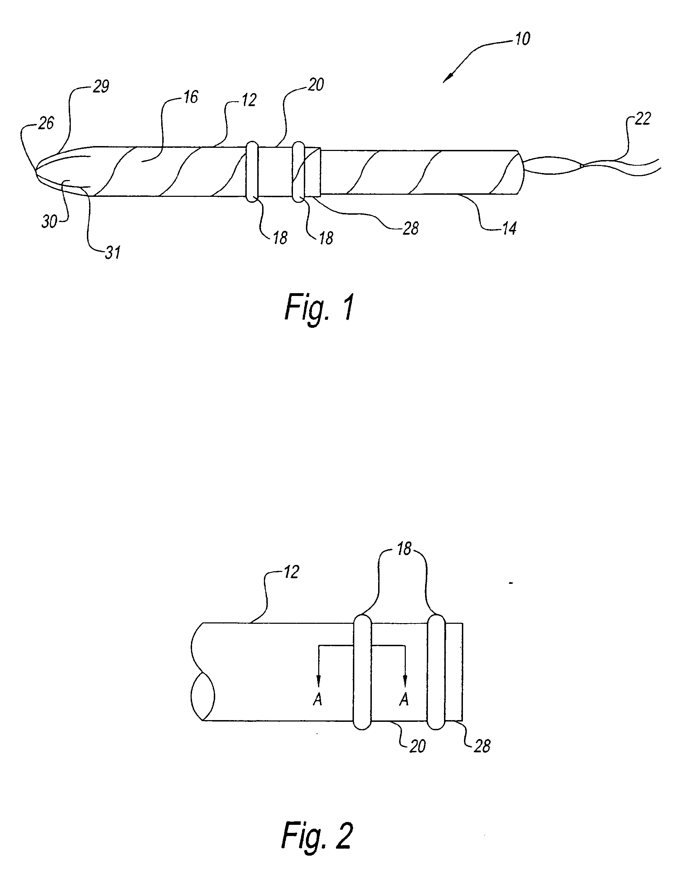

Referring to the drawings and, in particular, Fig. 1, there is shown a tampon applicator or inserter generally represented by reference numeral 10. Tampon applicator 10 houses and carries a tampon pledget (not shown) having a removal string 22. Tampon applicator 10 has a barrel 12 and a plunger 14 telescopically engageable with the barrel.

Barrel 12 has a central body 16 that is preferably tubular and can house and carry the pledget therein, a forward or ejection end 26, and an opposite, rear or plunger-receiving end 28. The plunger 1-4 can eject the pledget from barrel 12 out of ejection end 26 of the barrel into the vagina of a user.

Ejection end 26 of barrel 12 can be open or can have a substantially closed tip. The ejection end 26 preferably has a hemispherical, dome-shaped tip 29. The tip 29 may have a number of petals 30, which are preferably formed by a number of slits 31. The petals 30 are flexible, enabling the pledget to be ejected therethrough when plunger 14 is pressed against an end of the pledget within barrel 12.

Referring to Figs. 1 through 3, to improve grippability, barrel 12 preferably has one or more gripping structures 18. Preferably, the one or more gripping structures 18 are disposed on barrel 12 near plunger-receiving end 28. The one or more gripping structures 18 are preferably one or more embossments. The one or more embossments may be formed in any shape suitable for forming one or more gripping structures 18 on barrel 12. Preferably, the one or more gripping structures of the present invention are one or more embossed, ring-like structures.

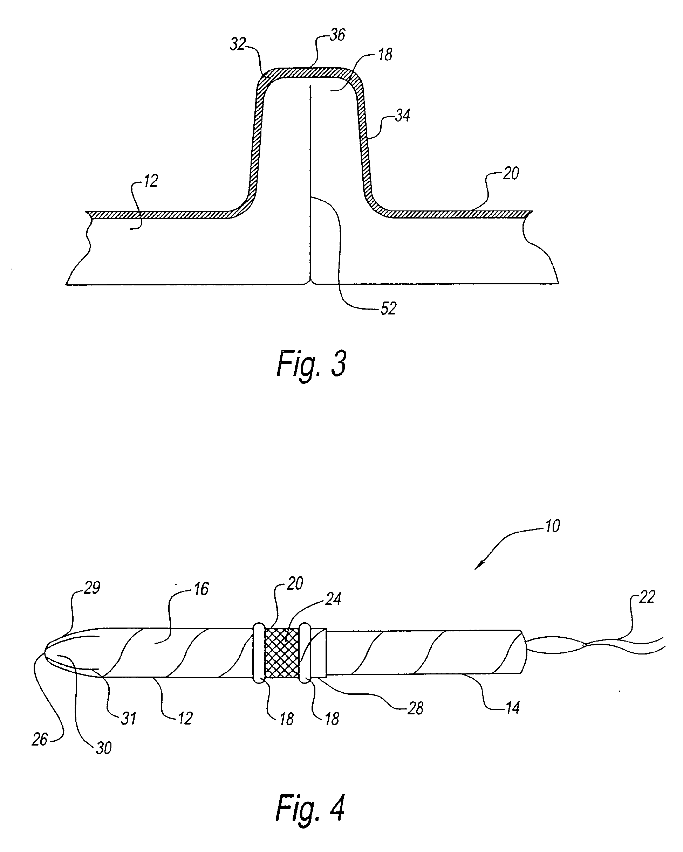

Referring to Fig. 3, the one or more embossed, ring-like structures have at least one acute edge 32 that improves grippability, thereby making for a more secure grip. The at least one acute edge 32 is formed by a substantially vertical sidewall 34 and a substantially planar top portion 36. It is this substantially vertical sidewall 34 that provides a user with an enhanced or superior grip on barrel 12 during use of applicator 10.

To provide the enhanced grippability, it has been found that the substantially vertical sidewall 34 is tapered, with respect to a perpendicular plane from the base of the sidewall, towards the substantially planar top portion about 0° to at most about 20°.

To further provide enhanced grippability of barrel 12, it has been found that the height of the one or more embossed ring-like structures is about 0.01 inches to about 0.1 inches, and preferably about 0.015 inches to about 0.06 inches. The width of the one or more embossed ring-like structures 18 is about 0.03 inches to about 0.1 inches, and preferably about 0.04 inches to about 0.05 inches.

Barrel 12 and/or plunger 14 of the present invention may be formed from any suitable material known in the art. Suitable materials include, but are not limited to, one or more biopolymers including polysaccharides and proteins, cardboard, heat shrink, paper, paper laminates, cardboard laminates, paper slurry, plastic, plastic tubing, pulp-molded paper, or any combinations thereof.

Additionally, barrel 12 and/or plunger 14 may be coated on the inside and/or the outside surface. The coating may be any suitable material to enhance strength, reduce surface friction, enhance aesthetics, or any combination thereof. Suitable coatings include, for example, cellophane, cellulose, epoxy, lacquer, nitrocellulose, nylon, plastic, polyester, polylactide, polyolefin, polyvinyl alcohol, polyvinyl chloride, silicone, wax, or any combinations thereof.

In another embodiment of the present invention, as depicted in Fig. 4, barrel 12 may also have one or more additional gripping structures 24 disposed in a finger-accepting gripping region 20 formed on barrel 12. Preferably, finger-accepting gripping region 20 is formed between two or more gripping structures 18. The one or more additional gripping structures 24 may be any structure suitable for increasing the grippability of barrel 12. The one or more additional gripping structures 24 may be smooth or, more preferably, may include one or more patterned or textured structures. The one or more additional gripping structures 24 may have an apex of the one or more gripping structures extend above and/or below the surface of barrel 12, or may be substantially aligned with the outer surface of barrel 12.

Suitable additional gripping structures 24 may include, but are not limited to, one or more abrasive materials, embossments, grooves, high wet coefficient of friction materials, lances, pressure sensitive adhesives, protuberances, slits, treads, or any combinations thereof. In addition, the one or more additional gripping structures 24 may be formed in any shape, including, but not limited to, arc, circle, concave, cone, convex, diamond, line, oval, polygon, rectangle, rib, square, triangle, or any combinations thereof.

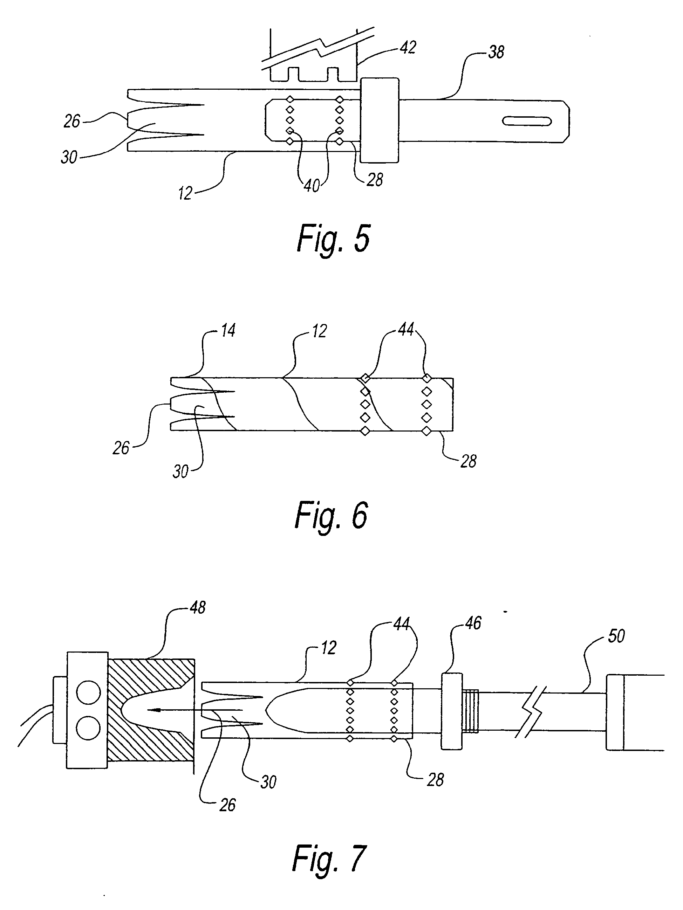

In another embodiment of the present invention, a novel method for forming the one or more embossed gripping structures is provided. Referring to Figs. 5 through 7, the method includes embossing barrel 12 such that a pattern of embossments is formed above the surface of the barrel to cause an axially weakened region. Preferably, at least one ring-like pattern is formed circumferentially around barrel 12. The embossing may be accomplished by any embossing process known to those skilled in the art.

In a preferred embodiment of the present invention, as depicted in Fig. 5, mandrel 38, having a punch-through crease pattern 40, is positioned in barrel 12, preferably from plunger-receiving end 28. With mandrel 38 and barrel 12 rotating, downward pressure is applied to the barrel by an upper wheel 42. As a result, a ring-like pattern of embossments 44 is formed on barrel 12, as depicted in Fig. 6.

Preferably, in one embodiment of the present invention, the ring-like pattern of embossments does not form a continuous band, but forms a series of individual embossments 44 aligned substantially in a circumferential row. In another preferred embodiment of the present invention, a petal tip, preferably a substantially closed petal tip, is formed on barrel 12 while forming the one or more embossed fingergrip structures.

Referring to Fig. 7, after the embossing, barrel 12 is placed over a petal-forming mandrel 46. Insertion end 26 of barrel 12 is then positioned in a petal former 48, to form petals 30 on barrel 12. At the same time that the petals 30 are formed, an axial force of sufficient magnitude is applied to barrel 12 at plunger-receiving end 28 to force some of the length of the barrel at the axially weakened region formed by the series of individual embossments to fold back upon themselves, like an accordion. This causes the formation of an outward protuberance, such as, for example, a ridge having at least one acute edge for gripping. It has been found that this invention not only provides one or more embossed gripping structures 18 having at least one acute edge for gripping, it also does not radially weaken barrel 12 at the one or more embossed gripping structures. Accordingly, the radial compressive strength of barrel 12 is not compromised. To the contrary, the radial compressive strength of barrel 12 may be increased since the one or more embossed gripping structures 18 act as stiffening structures.

Grippability can be further increased, if the individual embossments 44 are spaced apart at approximately 2 millimeters (mm) to about 20 mm, and preferably about 3 mm to about 6 mm. Wherever there is an individual embossment, applicator barrel 12 can be made to rupture during axial compression resulting in an acute point. The accordion-like fold between two adjacent embossments tends to be straight and does not exactly follow the radial curvature, if any, of barrel 12.

It should be understood that the fingergrip of the present invention could also be formed on an applicator barrel that has a non-circular cross-sectional fingergrip region, such as, for example, polygonal, flat, or oval cross sections.

If the embossed dots are placed closer together, the rupturing at the dots is less severe. Also, the one or more embossed ring-like gripping structures 18 appear less polygonal when viewed from the end. If the embossed dots are spaced further apart, the amount of rupturing does not significantly increase but the appearance tends to be less aesthetic since the one or more embossed ring-like gripping structures take on a distinct polygonal shape when viewed from the end.

The present invention has been described with particular reference to the preferred forms thereof. It will be obvious to one of ordinary skill in the art that changes and modifications may be made therein without departing from the spirit and scope of the present invention as defined by the following claims.

## Claims

1. A tampon applicator barrel comprising a body having at least one embossed gripping structure with at least one acute edge.

2. The tampon applicator barrel of claim 1, wherein the at least one acute edge is formed between a substantially planar top portion and a substantially vertical sidewall depending from the substantially planar top portion.

3. The tampon applicator of claim 2, wherein the substantially vertical side wall is tapered toward the substantially planar top portion from about 0° to about 20°.

4. The tampon applicator barrel of claim 1, wherein the at least one embossed gripping structure has a height about 0.01 inches to about 0.1 inches.

5. The tampon applicator barrel of claim 1, wherein the at least one embossed gripping structure has a width about 0.03 inches to about 0.1 inches.

6. The tampon applicator barrel of claim 1, wherein the body comprises at least two embossed gripping structures.

7. The tampon applicator barrel of claim 6, wherein the at least two embossed gripping structures are spaced such that at least one finger-accepting gripping surface is formed between the at least two embossed gripping structures.

8. The tampon applicator barrel of claim 7, wherein the at least one finger-accepting gripping surface comprises at least one additional gripping structure.

9. The tampon applicator barrel of claim 7, wherein the at least one additional gripping structure is selected from the group consisting of one or more abrasive materials, embossments, grooves, high wet coefficient of friction materials, lances, pressure sensitive adhesives, protuberances, slits, treads, and any combinations thereof.

10. The tampon applicator barrel of claim 7, wherein the at least one additional gripping structure is formed in one or more shapes selected from the group consisting of arc, circle, concave, cone, convex, diamond, line, oval, polygon, rectangle, rib, square, triangle, and any combinations thereof.

11. The tampon applicator barrel of claim 1, wherein the barrel is coated with a material selected from the group consisting of cellophane, cellulose, epoxy, lacquer, nitrocellulose, nylon, plastic, polyester, polyolefin, polyvinyl alcohol, polyvinyl chloride, silicone, wax, and any combinations thereof.

12. The tampon applicator barrel of claim 1, wherein the barrel is formed from a material selected from the group consisting of biopolymer, cardboard, heat shrink, paper, paper laminate, cardboard laminate, paper slurry, plastic, plastic tubing, pulp-molded paper, and any combinations thereof.
